Europäisches Patentamt

(19) European Patent Office

Office européen des brevets

(11) Publication number: **0 176 780**

**B1**

(12) **EUROPEAN PATENT SPECIFICATION**

(45) Date of publication of patent specification: **28.03.90**

(21) Application number: **85110983.5**

(22) Date of filing: **30.08.85**

(51) Int. Cl.⁵: **G 01 N 33/556**

(54) **A preservative solution for fixed avian erythrocytes for the viral hemagglutination test.**

(30) Priority: **31.08.84 JP 183039/84**

(43) Date of publication of application:
**09.04.86 Bulletin 86/15**

(45) Publication of the grant of the patent:
**28.03.90 Bulletin 90/13**

(84) Designated Contracting States:
**CH DE FR LI SE**

(56) References cited:
**DE-A-2 025 718**
**DE-B-1 617 467**
**DE-B-2 551 208**
**FR-A-2 416 471**
**GB-A-1 413 799**
**US-A-4 062 936**

(73) Proprietor: **SHIONOGI & CO., LTD.**
**1-8, Doshomachi 3-chome Chuo-ku**
**Osaka 541 (JP)**

(72) Inventor: **Sato, Akihiko**
**2-3-21, Minamiminohara**
**Ibaraki-shi Osaka (JP)**
Inventor: **Nakajima, Kunihiro**
**739-27, Toriya-cho**
**Kashihara-shi Nara (JP)**

(74) Representative: **Vossius & Partner**
**Siebertstrasse 4 P.O. Box 86 07 67**
**D-8000 München 86 (DE)**

Courier Press, Leamington Spa, England.

**Description**

Background of the invention
(1) Field of the invention

Some viruses carry on their surface a material called HA antigen (Hemagglutination antigen) which agglutinates animal erythrocytes. In the hemagglutination of HA antigen it is well known that a certain kind of viruses agglutinates erythrocytes of the corresponding kinds of animals. For instance, a rubella virus agglutinates erythrocytes of one-day-old chickens and geese, an influenza virus that of chickens (one-day-old and adult), a parainfluenza virus that of guinea pigs and chickens and a measles virus that of monkeys. Accordingly, viruses can be identified and quantified by utilizing the above property.

The present invention relates to a preservative solution for fixed erythrocytes employed in a hemagglutination reaction (HA reaction) and a hemagglutination inhibition reaction (HI reaction) based upon the above hemagglutination of viruses. The HA and HI reactions have generally been utilized in diagnosing viral diseases in the field of clinical tests.

(2) Description of the prior art

The HA reaction is employed in identifying a virus and determining its titer by utilizing the above specific antigenicity of several kinds of viruses and the HI reaction is utilized in determining an antibody against the HA antigen, that is, HI antibody which an organism acquires as a result of an immune response against a viral infection, and both reactions need erythrocytes as a reaction reagent. Though the use of fresh erythrocytes is preferred in order to make the agglutination or the agglutination inhibition reaction clear and accurate, this is practically impossible since it is not easy to get fresh erythrocytes for each test. Therefore a preservation method has been studied as the potency of erythrocytes for agglutination is not diminished even after long-term storage. Such preservation methods are e.g. fixation with formalin or freeze-drying of fixed erythrocytes. In the case of freeze-drying, a further stabilization of erythrocytes has been attempted by treatments before the freeze-drying such as washing which removes salt from the erythrocytes (JPN. Pat. No. 50-37724) or the addition of glucose or serum albumin (JPN. Unexamd. Pat. Publn. No. 54-23119).

FR—A—2 416 471 discloses a composition for the pretreatment of avian erythrocytes which are to be used in hemagglutination tests, consisting of water or a suitable buffer and kaolin particles. DE—A—2 025 718 discloses an erythrocyte preparation containing serum albumin and a sugar in addition to the erythrocytes which is suitable for lyophilization. GB—A—1 413 799 discloses a method for the preparation of a serological reagent wherein erythrocytes are treated with a suitable buffer solution which contains a preservative, e.g. sodium azide.

In developing a preservative solution able to keep erythrocytes stable for a long time without decreasing their ability to be agglutinated, we first intended to elucidate the cause of nonspecific inhibition of agglutination which restricts specific hemagglutination of the rubella virus and consequently makes its result inaccurate. As a result, it was found that the nonspecific inhibition of agglutination is caused by a phosphate buffer solution or a physiological salt solution used as a usual preservative solution for erythrocytes. The use of these solutions causes exudation of $\beta$-lipoprotein from the fixed erythrocytes to nonspecifically inhibit hemagglutination.

Therefore, if the fixed erythrocytes contain $\beta$-lipoprotein, in the rubella HI antibody titer test a negative specimen (antibody titer less than 8) may be misjudged as positive and accordingly a judgement based on the test becomes impossible.

In general, erythrocytes fixed with aldehydes can be preserved for a long time. They are, however, destroyed by mechanical stimulations such as shaking, mixing etc. and then $\beta$-lipoprotein exudes into an erythrocyte suspension. The reason why a great amount of $\beta$-lipoprotein exudes is deduced from the fact that, where fixed erythrocytes are preserved in a usual aqueous solution containing various salts such as physiological saline erythrocytes strongly adhere to each other under the influence of the salts and consequently they must be mechanically more stimulated for a long time in order to efficiently disperse them before use.

Summary

A preservative solution for fixed avian erythrocytes for the viral hemagglutination test of this invention comprises purified water such as distilled water or ion-exchanged water to which bovine serum albumin, glycerol or dimethyl sulfoxide are added. Additionally, as a preventive against the growth of bacteria sodium azide is added.

Where fixed avian erythrocytes are preserved in the preservative solution of this invention, the erythrocytes are well dispersed and stable after long-term cold storage, the adhesion of the fixed erythrocytes to each other is restricted and the exudation of $\beta$-lipoprotein is inhibited. Therefore the agglutination pattern is accurate and clear and thus the judgement of agglutination is easy. Since nonspecific inhibition of agglutination is restricted, precise HA titer and HI titer are given. This erythrocyte suspension provides a clear agglutination pattern in HA reaction and HI reaction of viruses such as the rubella virus, Japanese encephalitis virus, and mumps virus and does not alter after long-term storage in terms of the effect.

Brief description of the drawings

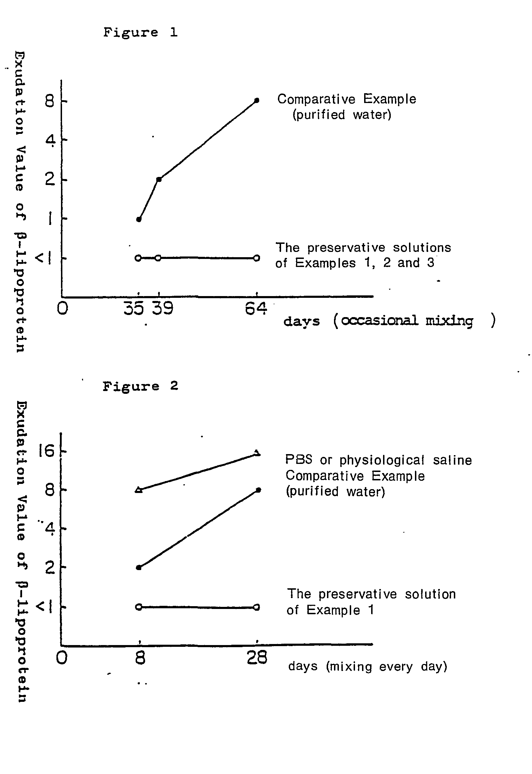

Figure 1 shows a change upon standing of the value of β-lipoprotein exuding in the erythrocyte suspension of the comparative example and each erythrocyte suspension using preservative solutions of Examples 1, 2 and 3. Figure 2 shows a change upon standing of the value of β-lipoprotein exuding in the erythrocyte suspension of the comparative example and each erythrocyte suspension using the preservative solution of Example 1, phosphate buffered saline (PBS) and physiological saline.

Detailed description of the invention

In order to prevent the above exudation of β-lipoprotein, it is necessary to suspend fixed erythrocytes in purified water, not in a phosphate buffer solution or physiological saline, and the nonspecific inhibition of agglutination can be prevented by this method.

Moreover, it was recognized that erythrocytes become more stable by the addition of an appropriate amount of bovine serum albumin, glycerol or methylsulfoxide to this preservative solution consisting of purified water and additionally, the addition of a preventive against the growth of bacteria makes erythrocytes more durable for long-term storage.

The fixed erythrocytes preservable in the solution of this invention are of avians such as geese or chickens and especially the effect on goose erythrocytes is remarkable. Fixation is achieved according to usual methods by using a usual fixative such as formaldehyde or glutaraldehyde. For example, erythrocytes are fixed with 0.1 to 0.3% (V/V) solution of glutaraldehyde in 1 to 2 hours.

The preservative solution of this invention comprises purified water such as distilled water or ion-exchanged water and either bovine serum albumin, glycerol or dimethyl sulfoxide. The bovine serum albumin is added in such a way that is concentration reaches 0.01 to 1.0% (W/V), preferably 0.05 to 0.2% (W/V). Glycerol or dimethyl sulfoxide are added in such a way that the concentration reaches from 5 to 10% (V/V) in the case of dimethylsulfoxide and 4 to 10% (V/V) in the case of glycerol.

Moreover, as a preventive against the growth of bacteria sodium azide is added whose concentration is as high as prepared for the purpose of antisepsis, i.e. 0.01 to 0.2% (W/V).

HA tests for viruses, especially the rubella virus, Japanese encephalitis virus, mumps virus and influenza virus, and HI tests for quantifying HI antibodies in sera can be performed by employing the above avian erythrocytes suspended in the preservative solution of this invention.

Where fixed avian erythrocytes are suspended in the preservative solution of this invention up to the desired concentration, preferably 8 to 12% (V/V), the erythrocytes are well dispersed and stable after long-term cold storage. Therefore the agglutination pattern is accurate and clear and thus the judgement of agglutination is easy. Since the nonspecific inhibition of agglutination is restricted, precise HA titer and HI titer are given. This erythrocye suspension provides clear agglutination patterns in HA reaction and HI reaction of viruses such as the rubella virus, Japanese encephalitis virus, and the mumps virus especially in those of the rubella virus, which do not alter after long-term storage in terms of the effect.

Example

This invention is exemplified by the following examples:

Comparative Example

100 ml of fixed goose erythrocytes are dispersed in purified water to give a 10% erythrocyte suspension.

Example 1

1 g of bovine serum albumin and 1 g of sodium azide are dissolved in purified water (total volume 1000 ml) under stirring for use in the preservation of fixed erythrocytes.

Example 2—6

The following ingredients and purified water (total volume 1000 ml) are mixed in the same way as in Example 1 to give a preservative solution.

# EP 0 176 780 B1

TABLE 1

| Example | Ingredient | Amount | Conc. |
|---------|------------|--------|-------|
| 2 | glycerol<br>sodium azide | 100 ml<br>1 g | 10% (V/V)<br>0.1% (W/V) |
| 3 | dimethyl sulfoxide<br>sodium azide | 100 ml<br>1 g | 10% (V/V)<br>0.1% (W/V) |
| 4 | bovine serum albumin<br>sodium azide | 0.5 g<br>0.5 g | 0.05% (W/V)<br>0.05% (W/V) |
| 5 | glycerol<br>sodium azide | 40 ml<br>1 g | 4% (V/V)<br>0.1% (W/V) |
| 6 | dimethyl sulfoxide<br>sodium azide | 56 ml<br>1.2 g· | 5.6% (V/V)<br>0.12% (W/V) |

Effect of the invention

(1) The fixed erythrocytes prepared in the following manner are suspended in the purified water of the comparative example and the preservative solution of Examples 1—3 to provide 10% suspensions, which are refrigerated under occasional stirring. The amount of exuded β-lipoprotein is quantified 35, 39 and 64 days later to give the results shown in Figure 1. In these preservative solutions the exudation of β-lipoprotein was not recognized.

Experimental method

a. Preparation of fixed goose erythrocytes

After washing the commercially available goose erythrocytes 3 times with physiological saline and once with phosphate buffered saline (PBS) containing the following composition, they are suspended in the same PBS to provide a 5% (V/V) suspension. To this erythrocyte suspension is added a 1.5% glutaraldehyde solution diluted with PBS at a rate of 1 part by volume per nine parts by volume of erythrocytes and the mixture is allowed to react at room temperature for 60 minutes by moderatetly stirring with a stirrer. The resulting fixed erythrocytes are washed 6 times with purified water and suspended in a preservative solution.

| | | |
|---|---|---|
| PBS: | potassium chloride | 0.2 g |
| | disodium hydrogenphosphate | 1.15 g |
| | potassium dihydrogenphosphate | 0.2 g |
| | sodium chloride | 8.0 g |
| | purified water up to | 1000 ml |

b. Method of quantification of lipoprotein

Lipoprotein is quantified through the ability to inhibit the hemagglutination of rubella HA antigen. 0.5 ml of a preservative solution in which fixed erythrocytes are well dispersed are transferred into a test tube and centrifuged, and the rubella HI test is performed with the resulting supernatant. In order to make the detection sensitivity of β-lipoprotein high, 2 units of rubella HA antigen are employed. The value of β-lipoprotein is defined as the reciprocal value of that dilution which is able to inhibit hemagglutination of the rubella virus. When the rubella HI test (according to the microplate method by the National Institute of Health, Tokyo, which comprises the use of VBS (Veronal buffer solution) containing BSA, gelatin, $Mg^{2+}$ and $Ca^{2+}$ as a dilution solution and goose or one-day-old chicken erythrocytes, the general procedure is e.g. disclosed in Stewart et al., N. Engl. J. Med. 276, 554 (1967) and Halonen et al, Proc. Soc. Exp. Biol. Med. 125, 167 (1967)) is performed with erythrocytes which exuded β-lipoprotein whose value was determined to be more than 8 by this method, negative serum is judged as positive.

(2) The fixed goose erythrocytes noted in (1) are suspended in the preservative solution of Example 1, PBS and physiological saline to give a 10% suspension. These suspensions and the suspension of comparative example are stored at 4—10°C with mixing once a day, and the value of exuded β-lipoprotein is determined 8 and 28 days after. The results are shown in Figure 2. β-lipoprotein exuding in the preservative solution of Example 1 was not detected.

(3) The rubella HA (antigen titer) and HI (antibody titer) tests on 48 clinical patient serum specimens were performed by using fixed goose erythrocytes suspended in the preservative solution of Example 1 and fresh erythrocytes suspended in a DGV (dextrose gelatin veronal) buffer solution according to the microplate method by NIH, Tokyo. As shown in Tables 2 and 3, the results of the case using fixed goose erythrocytes suspended in the preservative solution of this invention and the case using fresh erythrocytes were exactly the same.

4

TABLE 2
Comparison of rubella HA antigen titer

| Erythrocytes | Rubella HA titer (antigen titer) |
|---|---|
| fixed erythrocytes | 64 |
| fresh erythrocytes | 64 |

TABLE 3
Comparison of rubella HI antibody titer

Fixed erythrocytes in the preservative solution
HI antibody titer

| | <8 | 8 | 16 | 32 | 64 | 128 | 256 |
|---|---|---|---|---|---|---|---|
| 256 | | | | | | 1 | 3 |
| 128 | | | | | | 6 | 1 |
| 64 | | | | | 3 | | |
| 32 | | | | 7 | 1 | | |
| 16 | | 1 | 10 | 1 | | | |
| 8 | | 3 | | | | | |
| <8 | 11 | | | | | | |

HI antibody titer
Fresh erythrocytes in DGV buffer solution

**Claim**

A preservative solution of fixed avian erythrocytes for the viral hemagglutination test consisting either of 0.01 to 1.0% (W/V) of bovine serum albumin or 4 to 10% (V/V) of glycerol or 5 to 10% (W/V) of dimethyl sulfoxide and of 0.01 to 0.2% (W/V) sodium azide in aqueous solution.

**Patentanspruch**

Konservierungslösung für fixierte Vogelerythrocyten für den viralen Hämagglutinationstest, bestehend aus entweder 0,01 bis 1,0% (Gewicht/Volumen) Rinderserumalbumin oder 4 bis 10% (Volumen/Volumen) Glycerin oder 5 bis 10% (Gewicht/Volumen) Dimethylsulfoxid und 0,01 bis 0,2% (Gewicht/Volumen) Natriumazid in wäßriger Lösung.

**Revendication**

Solution préservatrice pour les erythrocytes d'oiseaux fixés pour l'essai de l'hémo-agglutination des virus consistant en soit 0,01 à 1,0% (P/V) d'albumine de sérum de bovin ou 4 à 10% (V/V) de glycérol ou 5 à 10% (P/V) de sulfoxyde diméthylique et de 0,01 à 0,2% (P/V) d'acide de sodium en solution aqueuse.

EP 0 176 780 B1

Figure 1

Exudation Value of β-lipoprotein

Comparative Example
(purified water)

The preservative solutions
of Examples 1, 2 and 3

days (occasional mixing )

Figure 2

Exudation Value of β-lipoprotein

PBS or physiological saline
Comparative Example
(purified water)

The preservative solution
of Example 1

days (mixing every day)

1